# EUROPEAN PATENT APPLICATION

(11) **EP 1 688 483 A1**
(43) Date of publication of application: **09.08.2006**
(21) Application number: 04748047.0
(22) Date of filing: 22.07.2004
(51) Int. Cl.: C12N 5/08, A61K 35/14

(54) **METHOD OF PREPARING LANGERHANS CELL FROM CD14-POSITIVE CELL BEING HUMAN PERIPHERAL-BLOOD MONONUCLEAR CELL WITH USE OF NOTCH LIGAND DELTA-1, GM-CSF AND TGF-BETA**

(30) Priority: 22.07.2003 JP 2003277892
(71) Applicant: KIRIN BEER KABUSHIKI KAISHA, Tokyo 104-8288 (JP)
(72) Inventor: KATAYAMA, Naoyuki, Tsu-shi, Mie 5140064 (JP); OISHI, Koshi, Tsu-shi, Mie 5142221 (JP); SHIKU, Hiroshi, Tsu-shi, Mie 5140061 (JP)
(74) Representative: HOFFMANN EITLE
(86) International application number: PCT/JP2004/010783
(87) International publication number: WO 2005/007839

(57) **Abstract**

This invention provides a method for preparing Langerhans cells from CD14-positive cells, which are human peripheral blood mononuclear cells. This method comprises culturing human peripheral blood mononuclear cells in the presence of Notch ligand, GM-CSF, and TGF-β.

## Description

### Technical Field

The present invention relates to culturing of epidermal Langerhans cells (LCs), which are members of a subtype of dendritic cells (DCs), and a method for preparing Langerhans cells from CD-14-positive cell in human peripheral blood mononuclear cells using Notch ligand Delta-1, granulocyte-macrophage colony-stimulating factor (GM-CSF), and transforming growth factor-β (TGF-β). Such cells can be used for therapeutic agents for cancers, infectious diseases, graft rejection, graft versus host disease, autoimmune diseases, allergic diseases, and the like.

### Background Art

Dendritic cells (DCs) are the most potent antigen-presenting cells that induce initial immune responses mediated by T cells (Steinman RM et al., Annu Rev Immunol 9: 271-296, 1991; Caux C. et al., Immunol Today 16: 2-5, 1995; Hart DNJ, Blood 90: 3245-3287, 1997; Cella MF et al., Curr Opin Immunol 9: 10-16, 1997; Banchereau J. et al., Nature 392: 245-252, 1998; Banchereau J. et al., Annu Rev Immunol 18: 767-811, 2000). On the surfaces of the epidermal Langerhans cells (LCs), which are members of a subtype of epidermal DCs, Langerin, E-cadherin, and CCR6 are expressed. These cells maintain Birbeck granules therein (Schuler G (ed.), Epidermal Langerhans Cells, CRC Press, Boca Raton, FL, 1991; Valladeau J. et al., Immunity 12: 71-81, 2000; Tang A. et al., Nature 361: 82-85, 1993; Greaves DR. et al., J Exp Med 186: 837-844, 1997). LCs adequately induce immune responses in response to a variety of foreign substances that penetrate the skin. Specifically, LCs take up foreign substances that have penetrated the skin, such as pathogens or tumor cells, migrate to regional lymph nodes, present the antigens processed therein to naive T cells or memory T cells, subject them to initial activation or reactivation, and effectively induce immune responses specific for antigens derived from the foreign substances. The activated T cells infiltrate the tissues invaded by foreign substances, accelerate the removal thereof, and protect tissues from excessive damage.

Through observation of allogeneic bone marrow transplantation for aplastic anemia, LCs in the skin of a patient who had received allogeneic bone marrow grafts were found to have expressed the same histocompatible antigen as that of the bone marrow donor. Accordingly, human LCs were considered to be derived from bone marrow cells (Volc-Platzer B. et al., N. Engl. J. Med, 310: 1123-1124, 1984). As a result of *in vitro* experiments in the past, it was reported that the culture of CD34-positive hemopoietic precursor cells in cord blood or peripheral blood in the presence of granulocyte-macrophage colony-stimulating factor (GM-CSF) and the tumor necrosis factor-α (TNF-α) results in differentiation of some of the cultured cells into LCs, indicating that LCs are derived from hematopoietic stem cells (Caux C. et al., Nature 360: 258-261, 1992; Caux C. et al., J Exp Med 184: 695-706, 1996; Strunk D. et al., Blood 87: 1292-1302, 1996). However, pathways of cell differentiation from hematopoietic stem cells into LCs and control mechanisms thereof have not yet been fully elucidated. It has also been reported that the culturing of human peripheral blood monocytes in the presence of hematopoietic factor combinations, such as GM-CSF, interleukin 4 (IL-4), and a transforming growth factor-β (TGF-β); GM-CSF and IL-15; or GM-CSF and TGF-β results in differentiation of some of the cells into LCs (Geissmann F. et al., J Exp Med 187: 961-966, 1998; Mohamadzadeh M. et al., J Exp Med 194: 1013-1020, 2001; Guironnet G. et al., J Leukoc Biol 71: 845-853, 2002). However, differentiation into LCs in the presence of these hematopoietic factors is not sufficient. Bone marrow-derived DCs different from LCs (i.e., dermal DCs) reside in the dermal layers of the skin, and differentiation of such dermal DCs from human peripheral blood monocytes is induced in the presence of GM-CSF and IL-4 (Romani N. et al., J Exp Med 180: 83-93, 1994; Sallusto F. et al., J Exp Med 179: 1109-1118, 1994). It is reported that Notch ligand Delta-1, which has been found to play a key role in the development or differentiation of various types of tissues including a hematopoietic system (Ohishi K. et al., Int J Hematol 75: 449-459, 2002; Ohishi K. et al., Semin Cell Dev Biol 14: 143-150, 2003), is associated with differentiation of dermal DCs from human peripheral blood monocytes or CD34-positive cells in bone marrow (Ohishi K. et al., Blood 98:1402-1407, 2001). Thus, it is deduced that differentiation of hematopoietic stem cells into DCs is controlled by a network composed of various types of cytokines or other factors.

### Disclosure of the invention

The present invention provides a method for preparing Langerhans cells from human peripheral blood mononuclear cells, i.e., CD14-positive cells.

In order to utilize Langerhans cells, which are members of a subtype of dendritic cells having antigen-presenting functions, for cell biological research or development of cell therapy techniques using DC-based vaccines, the present inventors have conducted concentrated studies concerning a method of culturing and differentiating such cells. It had been heretofore attempted to obtain Langerhans cells via culturing; however, cells that could be referred to as "Langerhans cells" had not been obtained in terms of the antigens expressed by the cells.

The present inventors cultured human peripheral blood mononuclear cells, i.e., CD14-positive cells, in a medium for culturing animal cells using Notch ligand, GM-CSF, and TGF-β, and found that Langerhans cells could be thereby obtained, which led to the completion of the method for preparing Langerhans cells according to the present invention.

Specifically, the present invention is as follows.
[1] A method for preparing a Langerhans cell, which comprises culturing a human peripheral blood mononuclear cell in the presence of Notch ligand, GM-CSF, and TGF-β.
[2] A method for preparing a Langerhans cell, which comprises transmitting Notch signaling from Notch of a human peripheral blood mononuclear cell using Notch ligand, and culturing the human peripheral blood mononuclear cell in the presence of GM-CSF and TGF-β.
[3] The method for preparing a Langerhans cell according to [1] or [2], which is characterized in that Notch ligand is immobilized on a culture vessel.
[4] The method for preparing a Langerhans cell according to [3], wherein Notch ligand constitutes a fusion peptide with another peptide, and an antibody reacting with the other peptide is immobilized on the culture vessel, so that Notch ligand is immobilized on the culture vessel via the bond between the antibody and the other peptide.
[5] The method for preparing a Langerhans cell according to [4], wherein the other peptide is myc.
[6] The method for preparing a Langerhans cell according to any one of [1] to [5], wherein Notch ligand is selected from the group consisting of Delta-1, Delta-2, Delta-3, Delta-4, Jagged-1, and Jagged-2.
[7] The method for preparing a Langerhans cell according to [6], wherein Notch ligand is Delta-1.
[8] The method for preparing a Langerhans cell according to [1] or [2], wherein the human peripheral blood mononuclear cell is an isolated CD14-positive cell.
[9] The method for preparing a Langerhans cell according to any one of [1] to [8], which is characterized in that E-cadherin, Langerin, and CCR6 are expressed on the surface of the Langerhans cell.
[10] The method for preparing a Langerhans cell according to [9], which is characterized in that MHC class I molecule HLA-ABC, MHC class II molecule HLA-DR, CD80, and CD86 are further expressed on the surface of the Langerhans cell.
[11] A method for preparing a Langerhans cell, which further comprises adding at least one member selected from the group consisting of CD40 ligand, TNF-α, and LPS to the Langerhans cell prepared by the method according to any one of [1] to [10], and culturing the obtained mixture.
[12] The method for preparing a Langerhans cell according to any one of [1] to [10], wherein the Langerhans cell is immature.
[13] The method for preparing a Langerhans cell according to any one of [1] to [11], wherein the Langerhans cell is mature.
[14] The Langerhans cell prepared by the method according to any one of [1] to [13].
[15] The Langerhans cell according to [14], on the surface of which E-cadherin, Langerin, and CCR6 are expressed.
[16] The Langerhans cell according to [15], on the surface of which MHC class I molecule HLA-ABC, MHC class II molecule HLA-DR, CD80, and CD86 are further expressed.
[17] A Langerhans cell derived from a human peripheral blood mononuclear cell, on the surface of which E-cadherin, Langerin, CCR6, MHC class I molecule HLA-ABC, MHC class II molecule HLA-DR, CD80, and CD86 are expressed.
[18] The Langerhans cell according to any one of [15] to [17], which is an immature cell.
[19] The Langerhans cell according to any one of [15] to [17], which is a mature cell.
[20] A pharmaceutical composition comprising the Langerhans cell according to any one of [14] to [19].
[21] The pharmaceutical composition according to [20], which is a therapeutic agent for cancers or infectious diseases.
[22] The pharmaceutical composition according to [21], wherein the Langerhans cell is a mature cell.
[23] The pharmaceutical composition according to [20], which is used for suppression of graft rejection after transplantation of cells, organs, or tissues; for treatment of graft versus host disease; and for treatment of autoimmune diseases or allergic diseases.
[24] The pharmaceutical composition according to [23], wherein the Langerhans cell is an immature cell.
[25] A method for preparing a Langerhans cell used for treatment of cancers or infectious diseases, which comprises culturing a peripheral blood mononuclear cell collected from a human in the presence of Notch ligand, GM-CSF, and TGF-β.
[26] A method for preparing a Langerhans cell used for suppression of cancers, infectious diseases, or graft rejection after transplantation of cells, organs, or tissues; for treatment of graft versus host disease; and for treatment of autoimmune diseases or allergic diseases; such method comprising culturing a peripheral blood mononuclear cell collected from a human in the presence of Notch ligand, GM-CSF, and TGF-β.
[27] The method for preparing a Langerhans cell used for treatment of the diseases according to [25] or [26], wherein Notch ligand is selected from the group consisting of Delta-1, Delta-2, Delta-3, Delta-4, Jagged-1, and Jagged-2.
[28] The method for preparing a Langerhans cell used for treatment of the diseases according to [27], wherein Notch ligand is Delta-1.

This description includes part or all of the contents as disclosed in the description and/or drawings of Japanese Patent Application No. 2003-277892, which is a priority document of the present application.

### Brief Description of Drawings

Fig. 1 shows the results of analyzing the phenotype of cells derived from CD14-positive cells using GM-CSF, IL-4, and TGF-β in the presence or absence of Delta-1.
Fig. 2 shows the results of analyzing the phenotype of cells derived from CD14-positive cells using GM-CSF and IL-15 in the presence or absence of Delta-1.
Fig. 3 shows the results of analyzing the phenotype of cells derived from CD14-positive cells using GM-CSF and TGF-β in the presence or absence of Delta-1.
Fig. 4 shows the results of analyzing the expression of E-cadherin and Langerin in LC-like CD1a-positive cells induced to differentiate by the addition of Delta-1, GM-CSF, and TGF-β, and the expression of E-cadherin and CCR6 in Langerin-positive cells induced by the addition of Delta-1, GM-CSF, and TGF-β.
Fig. 5 shows the results of analyzing the expression of molecules associated with antigen-presenting functions of cells induced to differentiate by the addition of Delta-1, GM-CSF, and TGF-β.
Fig. 6 shows the level of HES-1 gene expression enhanced by Delta-1 in CD14-positive cells.
Fig. 7 is an electron micrograph of cultured cells.
Fig. 8 shows the results of analyzing FITC-dextran uptake by LCs.
Fig. 9 shows the expressions of HLA-ABC, HLA-DR, CD80, CD86, CD40, CD54, and CD83 in LCs matured by CD40 ligand and TNF-α.
Fig. 10 shows activation of peptide-specific CD8-positive cells by mature LCs generated from CD14-positive cells.
Fig. 11 shows the results of stimulating autologous CD4-positive cells with mature LCs and mature DCs.

### Preferred Embodiments of the Invention

### 1. Preparation of Langerhans cells

The present invention provides a method for preparing Langerhans cells, which comprises culturing human peripheral blood mononuclear cells in the presence of Notch ligand, GM-CSF, and TGF-β.

Human peripheral blood mononuclear cells can be obtained from blood sampled from a human in accordance with a conventional technique. For example, peripheral blood is sampled from a human, and the sampled blood is subjected to density gradient centrifugation using Ficoll-Hypaque or the like to obtain such peripheral blood mononuclear cells. In the present invention, CD14-positive human peripheral blood mononuclear cells are used. Such CD14-positive cells can be isolated via a conventional technique with the use of, for example, MACS Microbead (Mitenyi Biotec). The purity of the CD14-positive cells used for preparing Langerhans cells is at least 90%, preferably at least 95%, and more preferably at least 98%. Whether or not the cells are monocytes can be determined via, for example, nonspecific esterase staining.

In the method of the present invention, the CD14-positive cells thus obtained from human peripheral blood mononuclear cells are processed with Notch ligand, GM-CSF, and TGF-β. The term "process" used herein means that CD14-positive cells are allowed to react with these compounds, and these compounds may be added to the CD14-positive cells at the time of culture thereof. In such a case, any of Notch ligand, GM-CSF, or TGF-β may be dissolved and added to a medium for culturing CD14-positive cells. Notch ligand, however, is preferably immobilized on the inner surface of a culture vessel or the like to be allowed to react with the cells, as described below.

Notch is a protein of a cell associated with the signal transmission associated with cell differentiation. Notch is activated upon the binding of Notch ligand thereto. Notch ligands are classified into Delta and Jagged. Delta has homologues referred to as Delta-1, Delta-2, Delta-3, and Delta-4, and Jagged has homologues referred to as Jagged-1 and Jagged-2 (Mumm J. S. et al., Dev. Biol., 228, 151-165, 2000). In the present invention, any of such Notch ligands can be used, and Notch ligands that may be discovered in the future may also be included. Delta-1 is particularly preferable. Notch ligand or DNA encoding the same can be isolated from Delta-1-expressing cells derived from a human, such as keratinocytes, via a gene amplification technique. Notch ligand or DNA encoding the same can also be synthesized based on existing sequence information (Gray GE. et al., Am. J. Pathol., 154, 785-793, 1999).

GM-CSF and TGF-β may be obtained from nature, or they may be recombinant products. GM-CSF and TGF-β can be readily prepared based on known gene sequence information, and commercial samples thereof may also be used.

A culture medium that is commonly used for culturing animal cells may be employed. Examples thereof include RPMI1640, DMEM, and MEM. According to need, animal serum such as FCS, sugar, amino acid, antibiotics, and the like are added to the medium. A serum-free medium or culture medium supplemented with human-derived serum, plasma, or its components instead of animal serum such as FCS is preferable. A culture vessel that is commonly used for cell culture may be used, and a form of culture vessel may be a plate, dish, flask, or the like in accordance with the scale of culture. When Notch ligand is immobilized on the inner surface of the culture vessel as described below, use of a culture vessel made of polystyrene, which is suitable for immobilization of proteins or the like, is preferable. Also, use of a culture vessel having adequate functional groups, such as amino groups, bound thereto is preferable. Alternatively, a blood-sampling bag may be used.

A culture system is not limited, and culture may be carried out in a batch or continuous system.

The density of CD14-positive cells obtained from human peripheral blood at the time of culture is 1 × 10⁴ to 1 × 10⁷ cells/ml, and preferably 1 × 10⁵ to 1 × 10⁶ cells/ml.

When culturing CD14-positive cells obtained from human peripheral blood, the GM-CSF concentration is preferably 10 to 100 ng/ml, and the TGF-β concentration is preferably 1 to 100 ng/ml.

Notch ligand is preferably immobilized on the inner surface of a culture vessel or the like as mentioned above. In addition, a notch ligand can also be immobilized on particles such as polystyrene beads, and such particles may be added to the culture system at the time of cell culture.

Notch ligand may be directly immobilized on the inner surface of a culture vessel or the like; however, it is preferable that Notch ligand be immobilized via a spacer. A spacer is not limited, but a peptide composed of several to a dozen amino acid residues is preferably used. Examples of peptides that can be used as spacers include myc protein, V5 protein, and polyhistidine such as 6×His. Such spacer is bound to Notch ligand, and a method of binding is not limited. DNA encoding such peptide may be fused in-frame with DNA encoding Notch ligand, and the resultant may be introduced into an adequate host cell and allowed to express as a recombinant fusion protein. Fusion of DNAs and production of gene recombinant proteins can be carried out in accordance with conventional techniques. These procedures can be carried out in accordance with methods disclosed in literature that is well known in the art, such as J. Sambrook, E. F. Fritsch & T. Maniatis, 1989: Molecular Cloning, a laboratory manual, second edition, Cold Spring Harbor Laboratory Press; and Ed Harlow and David Lanc, 1988: Antibodies, a laboratory manual, Cold Spring Harbor Laboratory Press. Even when other peptides are bound to Notch ligands, this procedure is referred to as a step of processing cells with Notch ligands in the present invention, as long as Notch ligands transmit Notch signaling to cells at the time of cell culture. The number of peptide molecules to be bound to a molecule of Notch ligand is not limited to 1. As the number of peptide molecules increases, the density of immobilized notch ligands is enhanced, and effects of Notch ligands are improved. A spacer composed of 1 to several tens of peptides, and preferably 1 to 15 peptides, is used.

Specific antibodies that are to be bound to the spacer peptides may be previously bound to the inner surface of the culture vessel, and the spacer peptides may be allowed to bind to antibodies to allow Notch ligands to indirectly bind to the inner surface of the culture vessel. Antibodies may be bound to the inner surface of the culture vessel via conventional techniques such as physical adsorption or chemical binding with the use of specific functional groups. The density of Notch ligands to be bound to the inner surface of the culture vessel is not limited. The binding properties vary depending on the type of the plate, bag, or the like to be employed, and thus, the density can be adequately determined. The higher the density of Notch ligands immobilized, the larger the effects of Notch ligands on cells. Accordingly, a higher density of immobilization is preferable. When culture is conducted using a polystyrene plate and allowing Notch ligand Delta-1 to be fused with 6 molecules of myc proteins, for example, anti-myc antibodies may be introduced into a culture vessel at a concentration of 1 µg/ml to 20 µg/ml and then immobilized.

CD14-positive cells obtained from human peripheral blood may be cultured for approximately 1 day to 10 days; for example, for 6 days in the presence of Notch ligand, GM-CSF, and TGF-β. Part of or the entire medium is exchanged during the culture according to need. In such a case, antigen expression on the surfaces of cultured cells may be inspected via FACS or other means to determine the adequate duration of culture to obtain Langerhans cells.

CD14-positive cells can be isolated from peripheral blood mononuclear cells thus obtained from human peripheral blood and cultured in the presence of Notch ligand, GM-CSF, and TGF-β to prepare Langerhans cells. In this case, processing of cells with Notch ligands results in binding of Notch ligands to Notch that the cells have inherently, which allows transmission of Notch signals to cells. Specifically, processing of cells with Notch ligands means that Notch expressed on cell is allowed to transmit Notch signals using Notch ligands.

Whether or not the obtained cells are Langerhans cells may be evaluated by inspecting the antigens on the cell surfaces. On the surfaces of the Langerhans cells prepared by the method of the present invention, E-cadherin, Langerin, and CCR6 are expressed. On the surfaces of the Langerhans cells prepared by the method of the present invention, MHC class I molecule HLA-ABC, MHC class II molecule HLA-DR, CD80, and CD86 are further expressed. Whether or not these antigens are expressed can be determined in the following manner. That is, antibodies reacting with such antigens that are labeled with a coloring enzyme, a fluorescent compound, or the like are employed to inspect whether or not the cells are stained, via microscopic observation or other means. For example, cells can be subjected to immunostaining with these antibodies to determine the presence or absence of antigens on the surfaces of the cells. Alternatively, the presence of antigens can be inspected with the use of magnetic beads having the aforementioned antibodies bound thereto. The presence of antigens on the cell surfaces can also be determined with the use of FACS or a flow cytometer. Examples of FACS or a flow cytometer that can be employed include FACS Vantage (Becton Dickinson) and FACS Calibur (Becton Dickinson).

According to the method of the present invention, mature and immature Langerhans cells can be obtained.

According to a culture method involving the presence of Notch ligand, GM-CSF, and TGF-β, immature Langerhans cells are primarily obtained, and the obtained immature Langerhans cells may be subjected to further maturation to obtain mature Langerhans cells. In order to stimulate the immune response system, use of mature Langerhans cells is preferable.

As factors that stimulate immature Langerhans cells to mature, factors that are commonly used for stimulating dendritic cells to mature can be used. Examples of such factors include, but are not limited to, TNF-α, LPS, IL-1 such as IL-1β, IL-6, prostaglandins such as PGE2; INF-α; INF-β; INF-γ; CD40 ligands; and adequate combinations thereof.

An adequate concentration of factors for permitting immature Langerhans cells to mature is, for example, 0.1 ng/ml to 100 µg/ml. For example, the TNF-α concentration is preferably 1 to 200 ng/ml, CD40 ligand concentration is preferably 0.1 to 10 µg/ml, and the LPS concentration is preferably 0.1 to 10 ng/ml.

The present invention also includes Langerhans cells obtained by culturing the human peripheral blood mononuclear cells in the presence of Notch ligand, GM-CSF, and TGF-β according to the method described above. On the surfaces of such Langerhans cells, antigens as mentioned above are expressed.

### 2. Applications of Langerhans cells

The present invention also include a pharmaceutical composition containing the Langerhans cells obtained by the above method. The Langerhans cells of the present invention can be applied to cell therapy, and a pharmaceutical composition comprising the Langerhans cells of the present invention can be used for cell therapy.

Such pharmaceutical composition can be used for treatment of cancers or infectious diseases such as AIDS; for suppression of graft rejection after transplantation of cells, organs, or tissues; for treatment of graft versus host disease; and for treatment of autoimmune diseases or allergic diseases. Mature Langerhans cells have immunopotentiating effects and thus are particularly suitable for treatment of cancers or infectious diseases such as AIDS. In contrast, immature Langerhans cells have immunosuppressive effects and thus are suitable for suppression of graft rejection after transplantation of cells, organs, or tissues; for treatment of graft versus host disease; and for treatment of autoimmune diseases or allergic diseases. The Langerhans cells, particularly immature Langerhans cells, of the present invention can be used for treatment of autoimmune diseases, such as chronic rheumatoid arthritis, multiple sclerosis, type I diabetes mellitus, iridocyclochoroiditis, autoimmune myocarditis, myasthenia gravis, systemic erythematodes, autoimmune hemolytic anemia, systemic scleroderma, ulcerative colitis, Crohn's disease, Sjogren's syndrome, autoimmune hepatopathy (e.g., primary biliary cirrhosis), psoriasis, idiopathic thrombocytopenic purpura, Goodpasture's syndrome (e.g., glomerular nephritis), pernicious anemia, Hashimoto's disease, vitiligo vulgaris, Behcet's disease, autoimmune gastritis, pemphigus, Guillain-Barre syndrome, and HTLV-1-associated myelopathy, or allergic diseases, such as contact hypersensitivity, allergic rhinitis, food allergy, and asthma.

When the Langerhans cells are used for treatment of cancers or infectious diseases, the Langerhans cells of the present invention may be permitted to mature ,and may then be used in that state. Alternatively, the Langerhans cells of the present invention may be cultured together with the peripheral blood of the patient, and the resulting cytotoxic T lymphocytes (CTLs) may be used.

Antigens that are adequately selected in accordance with the types of target diseases are applied to the Langerhans cells of the present invention. The antigen application may be continued for several days. In the case of protein antigens, 1 to 1,000 µg/ml, and preferably 10 to 100 µg/ml, of antigens may be applied *in vitro.*

When the pharmaceutical composition comprising the Langerhans cells of the present invention is used for treatment of diseases, such composition may be administered to a patient intravenously, subcutaneously, or intracutaneously at a dose of 0.5 × 10⁶ to 10⁹. Administration to a patient may be carried out as needed. In the case of graft rejection after transplantation of organs or tissues and graft versus host disease, it is particularly preferable that the pharmaceutical composition be administered to the patient prior to grafting, which would cause such symptoms. The timing and the dosage of the administration of Langerhans cells to a patient can be adequately determined in accordance with, for example, the type of disease, the severity of disease, and the conditions of the patient.

The present invention further provides a method for preparing Langerhans cells used for suppression of cancers, infectious diseases, or graft rejection after transplantation of cells, organs, or tissues; for treatment of graft versus host disease; and for treatment of autoimmune diseases or allergic diseases; such method comprising culturing peripheral blood mononuclear cells sampled from a human in the presence of Notch ligand, GM-CSF, and TGF-β. The Langerhans cells for treatment of diseases obtained by this method can be used for treating various types of diseases as mentioned above. In accordance with the purpose of treatment, cells obtained from the patient or cells obtained from other person can be adequately selected.

The present invention further provides a method for suppression of cancers, infectious diseases such as AIDS, or graft rejection after transplantation of cells, organs, or tissues; for treatment of graft versus host disease; and for treatment of autoimmune diseases or allergic diseases using the Langerhans cells.

Furthermore, the present invention concerns the use of the aforementioned human antigen-presenting cells in the production of pharmaceuticals for suppression of cancers, infectious diseases such as AIDS, or graft rejection after transplantation of cells, organs, or tissues; for treatment of graft versus host disease; and for treatment of autoimmune diseases or allergic diseases using Langerhans cells.

A list of references cited herein is provided below.
1) Steinman RM: The dendritic cell system and its role in immunogenicity, Annu Rev Immunol 9: 271-296, 1991
2) Caux C, Liu Y-J, Banchereau J: Recent advances in the study of dendritic cells and follicular dendritic cells, Immunol Today 16: 2-5, 1995
3) Hart DNJ: Dendritic cells: Unique leukocyte populations which control the primary immune response, Blood 90: 3245-3287, 1997
4) Cella MF, Sallusto F, Lanzavecchia A: Origin, maturation and antigen presenting function of dendritic cells, Curr Opin Immunol 9: 10-16, 1997
5) Banchereau J, Steinman RM: Dendritic cells and the control of immunity, Nature 392: 245-252, 1998
6) Banchereau J, Briere F, Caux C. Davoust J, Lebecque S, Liu Y-J, Pulendran B, Palucka K: Immunobiology of dendritic cells, Annu Rev Immunol 18: 767-811, 2000
7) Schuler G (ed.): Epidermal Langerhans Cells, CRC Press, Boca Raton, FL, 1991
8) Valladeau J, Ravel O, Dezutter-Dambuyant C, Moore K, Kleijmeer M, Liu Y, Duvert-Frances V, Vincent C, Schnitt D, Davoust J, Caux C, Lebecque S, Saeland S: Langerin, a novel C-type lectin specific to Langerhans cells, is an endocytic receptor that induces the formation of Birbeck granules, Immunity 12: 71-81, 2000
9) Tang A, Amagai M, Granger LG, Stanley JR, Udey MC: Adhesion of epidermal Langerhans cells to keratinocytes mediated by E-cadherin, Nature 361: 82-85, 1993
10) Greaves DR, Wang W, Dairaghi DJ, Dieu MC, Saint-Vis B, Franz-Bacon K, Rossi D, Caux C, McClanahan T, Gordon S, Zlotnik A, Schall TJ: CCR6, a CC chemokine receptor that interacts with macrophage inflammatory protein 3alpha and is highly expressed in human dendritic cells, J Exp Med 186: 837-844, 1997
11) Volc-Platzer B, Stingl G, Wolff K, Hinterberg W, Schnedl W: Cytogenetic identification of allogeneic epidermal Langerhans cells in a bone-marrow-graft recipient, N. Engl. J. Med 310: 1123-1124, 1984
12) Caux C, Dezutter-Dambuyant C, Schmitt D, Banchereau J: GM-CSF and TNF-α cooperate in the generation of dendritic Langerhans cells, Nature 360: 258-261, 1992
13) Caux C, Vanbervliet B, Massacrier C, Dezutter-Dambuyant C, de Saint-Vis B, Jacquet C, Yoneda K, Imamura S, Schmitt D, Banchereau J: CD34⁺ hematopoietic progenitors from human cord blood differentiate along two independent dendritic cell pathways in response to GM-CSF + TNFα, J Exp Med 184: 695-706, 1996
14) Strunk D, Rappersberger K, Egger C, Strobl H, Kromer E, Elbe A, Maurer D, Stingl G: Generation of human dendritic cells/Langerhans cells from circulating CD34⁺ hematopoietic progenitor cells, Blood 87: 1292-1302, 1996
15) Geissmann F, Prost C, Monnet JP, Dy M, Brousse N, Hermine O: Transforming growth factor β1, in the presence of granulocyte/macrophage colony-stimulating factor and interleukin 4, induces differentiation of human peripheral blood monocytes into dendritic Langerhans cells, J Exp Med 187: 961-966, 1998
16) Mohamadzadeh M, Berard F, Essert G, Chalouni C, Pulendran B, Davoust J, Bridges G, Palucka AK, Banchereau J: Interleukin 15 skews monocyte differentiation into dendritic cells with features of Langerhans cells, J Exp Med 194: 1013-1020, 2001
17) Guironnet G, Dezutter-Dambuyant C, Vincent C, Bechetoille N, Schmitt D, Peguet-Navarro J: Antagonistic effects of IL-4 and TGF-β1 on Langerhans cell-related antigen expression by human monocytes, J Leukoc Biol 71: 845-853, 2002
18) Romani N, Gruner S, Brang D, Kampgen E, Lenz A, Trockenbacher B, Konwalinka G, Fritsch PO, Steinman RM, Schuler G: Proliferating dendritic cell progenitors in human blood, J Exp Med 180: 83-93, 1994
19) Sallusto F, Lanzavecchia A: Efficient presentation of soluble antigen by cultured human dendritic cells is maintained by granulocyte/macrophage colony-stimulating factor plus interleukin 4 and downregulated by tumor necrosis factor α, J Exp Med 179: 1109-1118, 1994
20) Ohishi K, Varnum-Finney B, Bernstein ID: The notch pathway: Modulation of cell fate decisions in hematopoiesis, Int J Hematol 75: 449-459, 2002
21) Ohishi K, Katayama N, Shiku H, Varnum-Finney B, Bernstein ID: Notch signaling in hematopoiesis, Semin Cell Dev Biol 14: 143-150, 2003
22) Ohishi K, Varnum-Finney B, Serda RE, Anasetti C, Bernstein ID: The Notch ligand, Delta-1, inhibits the differentiation of monocytes into macrophages but permits their differentiation into dendritic cells, Blood 98:1402-1407, 2001
23) Ohishi K. Varnum-Finney B, Flowers D, Anasetti C, Myerson D. Bernstein ID: Monocytes express high amounts of Notch and undergo cytokine specific apoptosis following interaction with the Notch ligand, Delta-1, Blood 95: 2847-2854, 2000
24) Ohishi K, Varnum-Finney B, Bernstein ID: Delta-1 enhances marrow and thymus repopulating ability of human CD34⁺CD38⁻ cord blood cells, J Clin Invest 110: 1165-1174, 2002.
25) Mitani H, Katayama N, Araki H, Ohishi K, Kobayashi K, Suzuki H, Nishii K, Masuya M, Yasukawa K, Minami N, Shiku H: Activity of interleukin 6 in the differentiation of monocytes to macrophages and dendritic cells, Br J Haematol 109: 288-295, 2000
26) Araki H, Katayama N, Mitani H, Suzuki H, Nishikawa H, Masuya M, Ikuta Y, Hoshino N, Miyashita H, Nishii K, Minami N. Shiku H: Efficient *ex vivo* generation of dendritic cells from CD14⁺ blood monocytes in the presence of human serum albumin for use in clinical vaccine trials, Br J Haematol 114: 681-689, 2001
27) Inaba K, Inaba M, Romani N, Aya H, Deguchi M, Ikehara S, Muramatsu S, Steinman RM: Generation of large numbers of dendritic cells from mouse bone marrow cultures supplemented with granulocyte/macrophage colony-stimulating factor. J Exp Med 176, 1693-1702, 1992
28) Lowell S, Jones P, Le Roux I, Dunne J, Watt FM: Stimulation of human epidermal differentiation by delta-notch signaling at the boundaries of stem-cell clusters, Curr Biol 10: 491-500, 2000.
29) Valladeau J, Ravel O, Dezutter-Dambuyant C, Moore K, Kleijmeer M, Liu Y, Duvert-Frances V, Vincent C, Schmitt D, Davoust J, Caux C, Lebecque S, Saeland S: Langerin, a novel C-type lectin specific to Langerhans cells, is an endocytic receptor that induces the formation of Birbeck granules, Immunity 12: 71-81, 2000
30) Charbonnier AS, Kohrgruber N, Kriehuber E, Stingl G, Rot A, Maurer D: Macrophage inflammatory protein 3α is involved in the constitutive trafficking of epidermal langerhans cells, J Exp Med 190: 1755-1768, 1999
31) Luger TA, Bhardwaj RS, Grabbe S, Schwarz T: J Dermatol Sci. Regulation of the immune response by epidermal cytokines and neurohormones, J Dermatol Sci 13: 5-10, 1996
32) Larregina AT, Morelli AE, Spencer LA, Logar AJ, Watkins SC, Thomson AW, Falo LD Jr: Dermal-resident CD14⁺ cells differentiate into Langerhans cells, Nat Immunol 2: 1151-1158, 2001
33) Mumm JS, Kopan R: Notch signaling: from the outside in Dev Biol 228:151-165, 2000
34) Gray GE, Mann RS, Mitsiadis E, IIenrique D, Carcangiu ML, Banks A, Leiman J, Ward D, Ish-Horowitz D, Artavanis-Tsakonas S: Human ligands of the Notch receptor Am J Pathol 154:785-794, 1999

The present invention is hereafter described in greater detail with reference to the following examples, although the technical scope of the present invention is not limited thereto.

### Example 1: Preparation of Langerhans cells

The culture solutions and reagents used in the present example are described below.

RPMI 1640 (Nissui Pharmaceutical Co., Ltd., Tokyo) supplemented with 2 mM L-glutamine, 50 U/ml penicillin, 50 µg/ml streptomycin, and 10% fetal calf serum (FCS) (Hyclone, Logan, UT, U.S.A.) was used as culture medium. GM-CSF was provided by Kirin Brewery Co., Ltd. (Tokyo, Japan), and IL-4 was provided by Ono Pharmaceutical Co., Ltd. (Osaka, Japan). TGF-β and IL-15 were purchased from R&D Systems (Minneapolis, MN, U.S.A.). GM-CSF, IL-4, TGF-β, and IL-15 were each used at a concentration of 10 ng/ml.

Delta-1 was immobilized in the following manner.

Delta-1 was immobilized on a culture plate in accordance with a method that had been reported in the past (Ohishi K. et al., Blood 98: 1402-1407, 2001; Ohishi K. et al., Blood 95: 2847-2854, 2000; Ohishi K. et al., J Clin Invest 110: 1165-1174, 2002). At the outset, the concentration of the mouse anti-myc antibody F(ab')₂ fragment was adjusted to 5 to 10 µg/ml with PBS and the resultant was then applied to a 24-well tissue culture plate (Nunc, Roskilde, Denmark) at 37°C for 1 to 2 hours for immobilization. After the plate was washed with PBS, RPMI 1640 containing 2% to 10% FCS was applied to the culture plate at 37°C for 30 minutes in order to block nonspecific binding. Thereafter, a Delta-1 construct composed of the human Delta-1 extracellular domain and 6 myc proteins was applied and immobilized on the plate. The Delta-1 construct was purified from the culture supernatant of transfected NSO cell strains. As a control of the Delta-1 construct, a conditioned medium of untransfected NSO cell lines was used (Ohishi K. et al., Blood 98: 1402-1407, 2001; Ohishi K. et al., Blood 95: 2847-2854, 2000).

The antibodies used and their manufacturers are as follows.

Monoclonal antibodies used were anti-CD1a-FITC antibody and anti-HLA-ABC-PE antibody (DAKO, Glostrup, Denmark), anti-CD14-PE antibody, anti-CD80-PE antibody, and anti-HLA-DR-PE antibody (Becton Dickinson Immunocytometry, San Jose, CA, U.S.A.), anti-Langerin (CD207)-PE antibody and anti-E-cadherin antibody (Immunotech, Marseille, France), anti-CD86-PE antibody (Becton Dickinson Pharmingen, San Diego, CA, U.S.A.), anti-CCR6-FITC antibody (R&D Systems), mouse IgG2a-HTC antibody (Becton Dickinson), rat anti-mouse IgG1-FITC antibody and mouse IgG1-PE antibody (Becton Dickinson Pharmingen), and mouse IgG2b antibody (Coulter Miami, FL, U.S.A.).

The cells used in the present examples were isolated in the following manner.

After consent was obtained, peripheral blood was sampled from healthy Japanese volunteers using heparin-containing tubes. Peripheral blood mononuclear cells (PBMCs) were isolated via density gradient centrifugation using Ficoll-Hypaque (Nycomed Phama AS, Oslo, Norway). CD14-positive cells were isolated from PBMCs using MACS Microbeads (Miltenyi Biotec, Auburn, CA, U.S.A.) (Mitani H. et al., Br J Haematol 109: 288-295, 2000; Araki H. et al., Br J Haematol 114: 681-689, 2001). The purity of CD14-positive cells obtained in this manner was 95% or higher. The CD14-positive cells were subjected to nonspecific esterase staining (Muto Pure Chemicals Co., Ltd., Tokyo, Japan) and found to be positive for nonspecific esterase.

The obtained CD14-positive cells were cultured in the following manner.

CD14-positive cells were cultured on a 24-well plate for tissue culture at a seeding density of 5 × 10⁵ cells/ml using RPMI 1640 supplemented with 10% FCS. The culture medium was exchanged with fresh medium every 3 or 4 days. The cells were cultured for 6 days under such conditions and morphologically observed under a phase contrast microscope. The viable count was determined by the Trypan blue dye exclusion method.

Cellular phenotype was analyzed for evaluation in the following manner.

The surfaces of the cells were stained singly or dually with fluorescence-labeled or non-fluorescence-labeled monoclonal antibodies, and analyzed using the FACScan (Becton Dickinson Immunocytometry). The data was analyzed using CellQuest software (Becton Dickinson Immunocytometry). The cells were incubated to block nonspecific binding of the cells with 2% AB serum and then stained with various types of antibodies at 4°C for 30 minutes. Propium iodide-positive cells were eliminated as dead cells. In dual staining with E-cadherin and Langerin, the cells were first allowed to react with the anti-E-cadherin antibody, the resulting cells were washed, rat-anti-mouse IgG1-FITC antibody was applied thereto, nonspecific binding was blocked with a sufficient amount of the mouse IgG1 antibody, and the cells were then stained with anti-Langerin-PE antibody.

### (1) Effects of Delta-1 on CD14-positive cells in the presence of GM-CSF, IL-4, and TGF-β

In order to examine the effects of Delta-1 on the differentiation of human peripheral blood monocytes into LCs, the CD14-positive cells separated from PBMCs were cultured on a culture plate for 6 days, in which Delta-1 had been immobilized in advance, in the presence of GM-CSF, IL-4, and TGF-β (Geissmann F. et al., J Exp Med 187: 961-966, 1998), GM-CSF and IL-15 (Mohamadzadeh M. et al., J Exp Med 194: 1013-1020, 2001), or GM-CSF and TGF-β (Guironnet G. et al., J Leukoc Biol 71: 845-853, 2002), which have been reported to assist in the differentiation of monocytes into LCs. Cell morphology and phenotypes were then examined. As a control for Delta-1, the conditioned medium described above was used. The CD14-positive cells were cultured on a 24-well plate at a seeding density of 5 × 10⁵ cells/ml in the presence or absence of Delta-1 with the addition of GM-CSF, IL-4, and TGF-β. Cells were collected on day 6 of culture and then stained with the monoclonal antibodies reacting with the antigen molecules shown in the drawing.

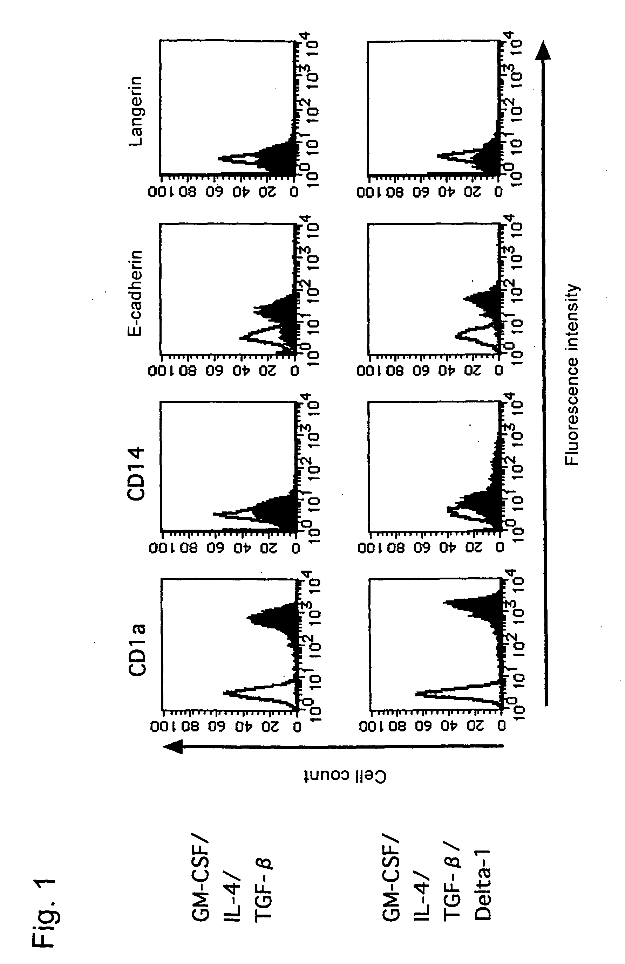

Fig. 1 shows the results of analyzing the phenotype of cells derived from CD14-positive cells using GM-CSF, IL-4, and TGF-β in the presence or absence of Delta-1.

The cells induced to differentiate by the addition of GM-CSF, IL-4, and TGF-β were morphologically DC-like cells. These cells were CD1a-positive and CD14-negative, and E-cadherin was expressed but Langerin was not expressed. Although no morphological change was observed with the addition of Delta-1, the level of E-cadherin expression was slightly enhanced (Fig. 1). In Fig. 1, the outlined portions indicate the results of analysis with the use of control antibodies, and the silhouette portions indicate the results of analysis with the use of the monoclonal antibodies against described antigens. The results were representative of several experiments.

### (2) Effects of Delta-1 on CD14-positive cells in the presence of GM-CSF and IL-15

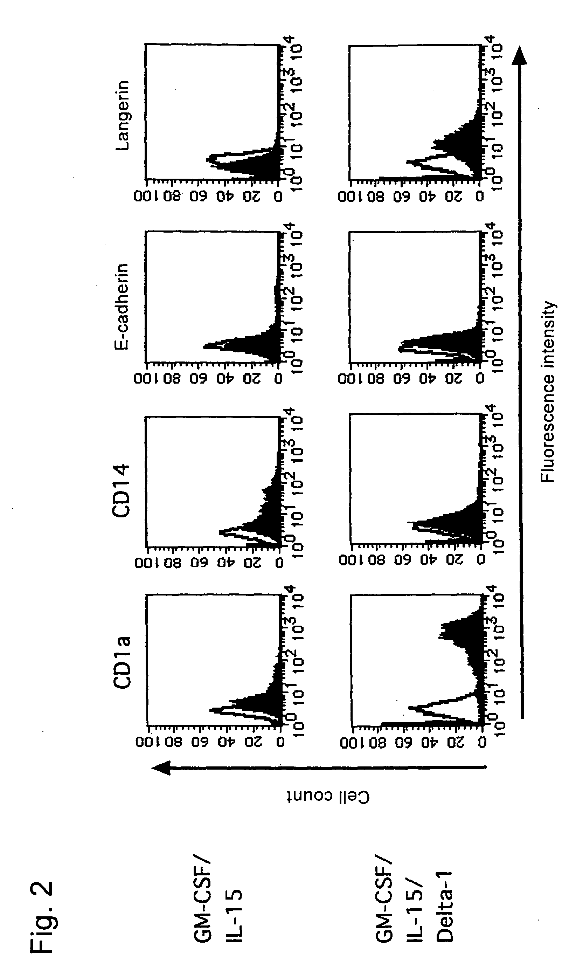

Subsequently, the effects of Delta-1 on the CD14-positive cells in the presence of GM-CSF and IL-15 were examined. The CD14-positive cells were cultured on a 24-well plate at a seeding density of 5 × 10⁵ cells/ml in the presence or absence of Delta-1 with the addition of GM-CSF and IL-15. Cells were collected on day 6 of culture and then stained with the monoclonal antibodies reacting with the antigen molecules shown in the drawing. The results are shown in Fig. 2. The outlined portions indicate the results of analysis with the use of control antibodies, and the silhouette portions indicate the results of analysis with the use of the monoclonal antibodies against described antigens. The results were representative of several experiments. The cells induced to differentiate by the addition of GM-CSF and IL-15 were morphologically macrophage-like cells. The surfaces of these cells were CD1a-weak-positive and CD14-weak-positive, and these cells were E-cadherin- and Langerin-negative. Although the expression of CD1a was induced and that of Langerin was slightly induced with the addition of Delta-1, expression of E-cadherin was not induced.

### (3) Effects of Delta-1 on CD14-positive cells in the presence of GM-CSF and TGF-β

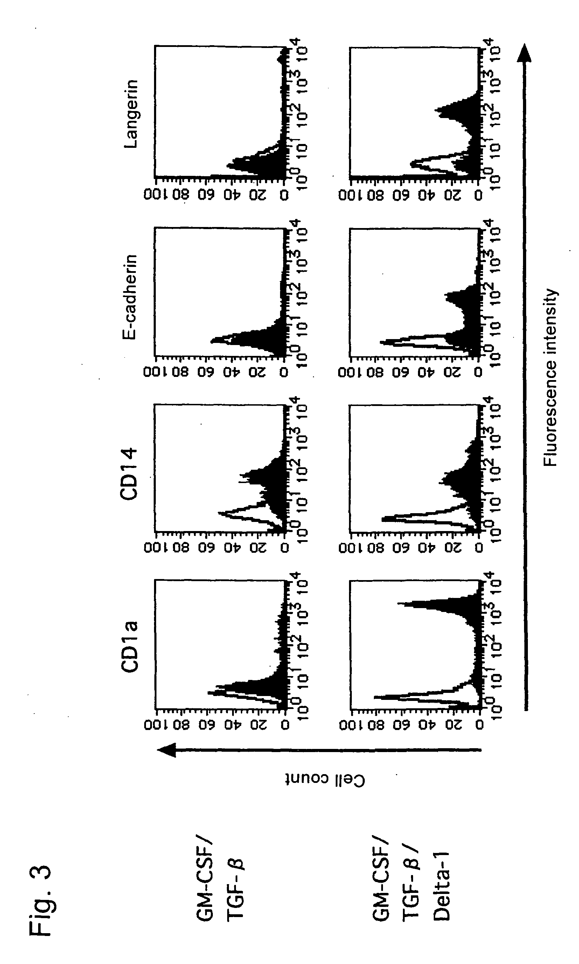

Effects of Delta-1 on the CD14-positive cells in the presence of GM-CSF and TGF-β were also examined. The CD14-positive cells were cultured on a 24-well plate at a seeding density of 5 × 10⁵ cells/ml in the presence or absence of Delta-1 with the addition of GM-CSF and TGF-β. Cells were collected on day 6 of culture and then stained with the monoclonal antibodies reacting with the antigen molecules shown in the drawing. The results are shown in Fig. 3. The outlined portions indicate the results of analysis with the use of control antibodies, and the silhouette portions indicate the results of analysis with the use of the monoclonal antibodies against described antigens. The results were representative of several experiments. The cells induced to differentiate by the addition of GM-CSF and TGF-β were morphologically macrophage-like cells. These cells were CD1a-negative, CD14-positive, E-cadherin-negative, and Langerin-negative. While the expression of CD 14 was maintained with the addition of Delta-1, expression of CD1a was strongly induced, and these cells were E-cadherin- and Langerin-positive.

### (4) Expression of E-cadherin and Langerin in LC-like CD1a-positive cells induced to differentiate by the addition of Delta-1, GM-CSF, and TGF-β and expression of E-cadherin and CCR6 in Langerin-positive cells

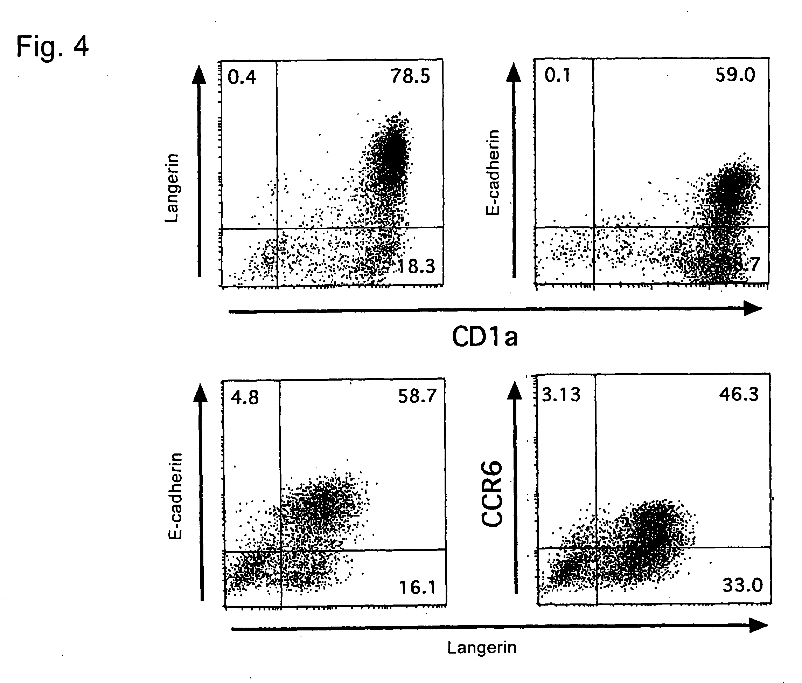

In order to determine that cells induced to differentiate by the addition of Delta-1, GM-CSF, and TGF-β were LCs, the expression of E-cadherin and Langerin in CD1a-positive cells and the expression of E-cadherin and CCR6 in Langerin-positive cells were examined. The CD14-positive cells were cultured on a 24-well plate at a seeding density of 5 × 10⁵ cells/ml with the addition of Delta-1, GM-CSF, and TGF-β. Cells were collected on day 6 of culture and then stained with the anti-CD1a antibody and the anti-Langerin or anti-E-cadherin antibody or with the anti-Langerin antibody and the anti-E-cadherin or anti-CCR6 antibody. The results of analysis are shown by dot plotting in Fig. 4. The results were representative of several experiments. Among the CD1a-positive cells, 81% thereof were Langerin-positive and 60% thereof were E-cadherin-positive. Among the Langerin-positive cells, 78% thereof were E-cadherin-positive, and 58% thereof were CCR6-positive. From 5 × 10⁵ CD14-positive cells, 2.1 ± 0.7 × 10⁵ (n = 5) cells were collected.

### (5) Expression of molecules associated with antigen-presentation of LC-like cells induced to differentiate by the addition of Delta-1, GM-CSF, and TGF-β

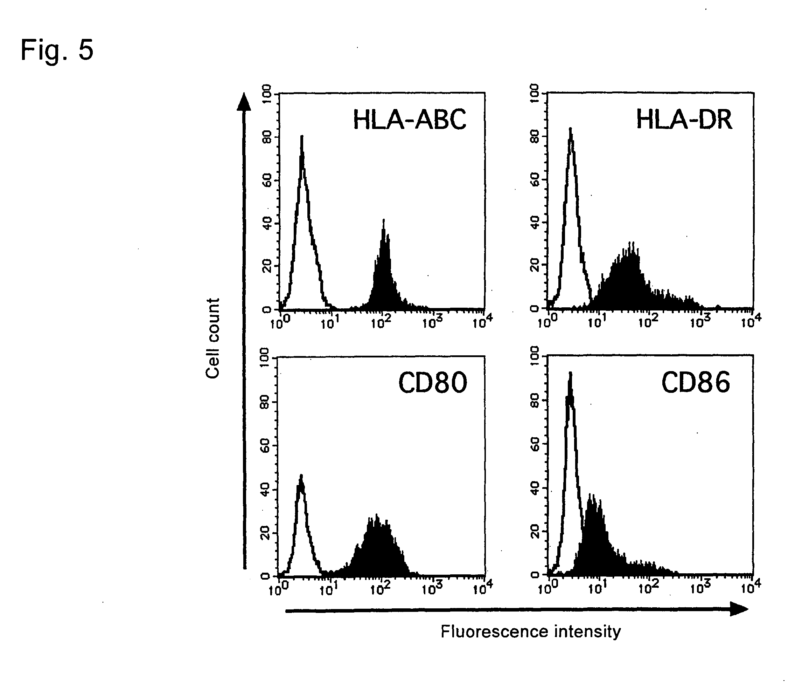

The expression of molecules associated with antigen-presentation of LC-like cells induced to differentiate by the addition of Delta-1, GM-CSF, and TGF-β was examined using a Langerin-positive cell fraction. The CD14-positive cells were cultured on a 24-well plate at a seeding density of 5 × 10⁵ cells/ml with the addition of Delta-1, GM-CSF, and TGF-β. Cells were collected on day 6 of culture and then stained with the anti-Langerin antibody and with the monoclonal antibodies reacting with the antigen molecules shown in the drawing. The expression of antigen molecules in the Langerin-positive cells were analyzed. The results are shown in Fig. 5. The outlined portions indicate the results of analysis with the use of control antibodies, and the silhouette portions indicate the results of analysis with the use of the monoclonal antibodies against described antigens. The results were representative of several experiments. In the cells in the Langerin-positive fraction, the major histocompatibility complex (MHC) class I and class II molecules, HLA-ABC and HLA-DR, and the co-stimulatory molecules CD80 and CD86 were expressed.

The following was found based on examinations (1) to (5) above.

DCs reside in the epidermis and in the dermis of the skin are referred to as LCs and dermal DCs, respectively (Steinman RM. et al., Annu Rev Immunol 9: 271-296, 1991; Banchereau J. et al., Annu Rev Immunol 18: 767-811, 2000). In the past, DCs were considered to be monocyte/macrophage lineage cells. Since DCs were found to reside in the granulocyte-macrophage colony that is generated in the presence of GM-CSF in the system for culturing mouse bone marrow cells, DCs were confirmed to be monocyte/macrophage lineage cells (Inaba K. et al., J Exp Med 176: 1693-1702, 1992). Thereafter, the *in vitro* culture system enabled differentiation of LCs from CD34-positive cells of human cord blood or peripheral blood or that of dermal DCs from CD14-positive cells of the peripheral blood. Thus, cell biology concerning human myeloid DCs advanced remarkably, and understanding thereof became further developed (Caux C. et al., Nature 360: 258-261, 1992; Caux C. et al., J Exp Med 184: 695-706, 1996; Strunk D. et al., Blood 87: 1292-1302, 1996; Geissmann F. et al., J Exp Med 187: 961-966, 1998; Mohamadzadeh M. et al., J Exp Med 194: 1013-1020, 2001; Guironnet G. et al., J Leukoc Biol 71: 845-853, 2002). Through observation of the culture system with the elapse of time, LCs were recognized as cells differentiated from hemopoietic precursor cells through CD 1a-positive CD 14-negative cells. LCs were considered to be of a cell group of a different lineage than that of the CD14-positive monocyte/macrophage cells (Caux C. et al., J Exp Med 184: 695-706, 1996). However, it was reported that LC-like cells were differentiated from CD14-positive monocytes of peripheral blood with the aid of a combination of several cytokines, and a possibility of another pathway of differentiation into LCs was presented (Geissmann F. et al., J Exp Med 187: 961-966, 1998; Mohamadzadeh M. et al., J Exp Med 194: 1013-1020, 2001; Guironnet G. et al., Dezutter-Dambuyant C. et al., J Leukoc Biol 71: 845-853, 2002).

LCs reside in the epidermis with keratinocytes, and Delta-1, which affects differentiation of CD14-positive monocytes or dermal DC, is expressed in keratinocytes (Ohishi K. et al., Blood 98: 1402-1407, 2001; Ohishi K. et al., Blood 95: 2847-2854, 2000; Lowell S. et al., Curr Biol 10: 491-500, 2000). Based on the above conditions, the present inventors studied the effects of Delta-1 in the culture system where LCs are differentiated from CD14-positive monocytes, which have been reported in the past. When CD14-positive monocytes were cultured in the presence of GM-CSF, IL-4, and TGF-β, or GM-CSF, IL-4, TGF-β, and Delta-1, E-cadherin was expressed in the resulting cells, although Langerin was not expressed at all. Langerin, which is the C-type lectin, is a molecule closely related to the Birbeck granule and has high specificity for LCs (Valladeau J. et al., Immunity 12: 71-81, 2000). Thus, cells in which such molecule is not expressed are not considered to have sufficient properties of LCs. Cells obtained in the presence of GM-CSF and IL-15 were E-cadherin- and Langerin-negative. In the cells obtained via culturing in the presence of Delta-1 in addition to GM-CSF + IL-15, a slight level of Langerin expression was observed, but E-cadherin was not expressed. Thus, none of these cells were considered to have properties of LCs. The cells obtained via culturing in the presence of GM-CSF and TGF-β were Langerin- and E-cadherin-negative. Thus, the same conclusion as the aforementioned would apply to such cells. As of the time of filing of the present invention, differences between the past reports (Geissmann F. et al., J Exp Med 187: 961-966, 1998; Mohamadzadeh M. et al., J Exp Med 194: 1013-1020, 2001; Guironnet G. et al., J Leukoc Biol 71: 845-853, 2002) and the results of experiments conducted by the present inventors cannot be explained. The present inventors thus consider that further studies are necessary in this regard. In the culture system containing Delta-1 in addition to GM-CSF and TGF-β, expression of E-cadherin and Langerin was observed in 50% or more of the cells, and E-cadherin was expressed in a considerable number of Langerin-positive cells. Further, approximately half of the Langerin-positive cells expressed CCR6 that is the receptor for the chemokine macrophage inhibitory protein-3α (MIP-3α), which is said to be expressed in immature LCs and associated with migration thereof to the dermis (Charbonnier AS. et al., J Exp Med 190: 1755-1768, 1999). These results indicate that cells differentiated from CD14-positive monocytes in the presence of GM-CSF, TGF-β, and Delta-1 were LCs. The fact that human MHC class I molecule HLA-ABC, MHC class II molecule HLA-DR, and co-stimulatory molecules CD80 and CD86 were expressed in these cells indicates that these cells have antigen-presenting functions.

Keratinocytes not only express Delta-1 (Lowell S. et al., Curr Biol 10: 491-500, 2000) but also secrete GM-CSF and TGF-β (Luger TA. et al., J Dermatol Sci 13: 5-10, 1996). Specifically, the culture system presented by the present inventors, in which LCs were differentiated from CD14-positive monocytes *in vitro,* was considered to involve conditions similar to *in vivo* skin conditions. Thus, it can be deduced that CD14-positive monocytes of the peripheral blood penetrate the epidermis through the dermis in a living organism, they are brought into contact with GM-CSF, TGF-β, or Delta-1 secreted from or expressed on keratinocytes, and then the CD14-positive monocytes are differentiated into LCs. There are reported dermal resident CD14-positive LC precursor cells that are different from monocytes/macrophage cells in terms of, for example, adhesiveness, phenotypes, or reactivity with the macrophage colony-stimulating factor (Larregina AT. et al., Nat Immunol 2: 1151-1158, 2001). The LC-like cells, which are differentiated from CD14-positive cells by the present inventors, are also CD14-positive, and the correlation between these two cell groups will draw attention.

### Example 2: Analysis of properties of prepared Langerhans cells

The reagents and the antibodies used in Example 2 are as follows.

CD40 ligand was purchased from Bender MedSystems (Vienna, Austria). TNF-α was provided by Dainippon Pharmaceutical Co., Ltd. (Suita, Japan), and IL-2 was provided by Takeda Chemical Industries, Ltd. (Osaka, Japan). CD40 ligand was used at 1 µg/ml, TNF-α was used at 20 ng/ml, and IL-2 was used at 20 IU/ml. The monoclonal antibodies used were anti-CD40-PE antibody and anti-CD83-PE antibody (Immunotech, Marseille, France), and anti-CD54-PE antibody (Becton Dickinson Immunocytometry, San Jose, CA, U.S.A.).

Cells were isolated from peripheral blood in the following manner. After consent was obtained, peripheral blood was sampled from healthy Japanese volunteers using heparin-containing tubes. PBMCs were isolated via density gradient centrifugation using Ficoll-Hypaque (Nycomed Phama AS, Oslo, Norway). CD8-positive cells and CD4-positive cells were isolated from PBMCs using MACS Microbeads (Miltenyi Biotec, Auburn, CA, U.S.A.). The purity of CD8-positive cells and that of CD4-positive cells obtained in this manner were both 99% or higher.

Real-time RT-PCR (real-time reversed transcriptase-polymerase chain reaction) was carried out in accordance with a method that had already been reported (Ohishi K, J Clin Invest 110: 1165-1174, 2002). Immediately after isolation, the CD14-positive cells were cultured in the presence of Delta-1, GM-CSF, and TGF-β for 24 hours, total RNA was extracted from these cells, and cDNA was then synthesized. The primers used are as follows: the HES-1 forward primer: 5' TGG AAA TGA CAG TGA AGC ACC 3' (SEQ ID NO: 1); the HES-1 reverse primer: 5' GTT CAT GCA CTC GCT GAA GC 3' (SEQ ID NO: 2); the HES-1 probe: 5' (FAM)-CGC AGA TGA CGG CTG CGC TG-(TAMRA) 3' (SEQ ID NO: 3); the GAPDH endogenous gene forward primer: 5' GAA GGT GAA GGT CGG AGT 3' (SEQ ID NO: 4); the GAPDH reverse primer: 5' GAA GAT GGT GAT GGG ATT TC 3' (SEQ ID NO: 5); and the GAPDH probe: 5' (FAM)-TTG CCA TCA ATG ACC CCT TCA TTG AC-(TAMRA) 3' (SEQ ID NO: 6). Amplification was carried out using ABI Prism 7,700 Sequence Detector (Applied Biosystems, Foster City, CA, U.S.A.).

The CD14-positive cells were cultured in the presence of Delta-1, GM-CSF, and TGF-β for 6 days, immobilized with 2.5% glutalaldehyde, dehydrated with ethanol, and then embedded in epon. The sections were stained with lead citrate and uranyl acetate, and the cells were then observed under a JEM 1200EX electron microscope (JEOL, Tokyo).

The phagocytic activity of the differentiated LCs was assayed in the following manner. Culture was conducted in the presence of Delta-1, GM-CSF, and TGF-β for 4 days, CD40 ligands and TNF-α were further added thereto, and culture was continued for an additional 2 days. The resulting LCs (2 x 10⁵ cells) were suspended in 10% FCS RPMI 1640, and the resultant was allowed to react with 1 mg/ml FITC-dextran (molecular weight: 40,000; Sigma, St. Louis, MO, U.S.A.) on ice or at 37°C for 1 hour. FITC-dextran uptake was terminated with cooled 1% FCS PBS, and the cells were then washed 4 times with 1% FCS PBS. FITC-dextran uptake was assayed using a FACS Calibur.

An enzyme-linked immunosorbent spot (ELISPOT) assay was carried out while making minor modifications in the method that had been reported in the past (Ikuta Y et al., Blood 99: 3717-3724, 2002). CD14-positive cells were cultured in the presence of Delta-1, GM-CSF, and TGF-β for 6 days, and during the last 2 days thereof culture was conducted in the presence of CD40 ligands and TNF-α to prepare mature LCs. The anti-interferon-γ-antibody (10 µg/ml, 1-D1K; Mabtech, Stockholm, Sweden) was added to a 96-well nitrocellulose MAHA S4510 Millipore plate (Millipore, Bedford, MA, U.S.A.) and allowed to stand at 4°C overnight. This plate was washed with PBS and then treated with 10% serum of type AB at 37°C for 2 hours in order to block nonspecific binding. Mature LCs were pulsed with modified Melan-A₂₆₋₃₅ peptides (A27L) (ELAGIGILTV SEQ ID NO: 7). CD8-positive cells (2 x 10⁵ cells) obtained from healthy HLA-A0201-positive volunteers were stimulated with, irradiated (46Gy), peptide-pulsed mature LCs in RPMI 1640 containing 10% AB serum on a 24-well plate. IL-2 (20 IU/ml) was added thereto on days 4 and 7 of culture. These CD8-positive cells were collected on day 10 of culture and used as effector cells. T2 cells that had been pulsed with the modified Melan-A₂₆₋₃₅ peptides (A27L) or T2 cells that had not been pulsed were employed as target cells. The effector cells (1 x 10⁴ cells) were subjected to culture with the target cells (5 x 10⁴ cells) on the Elispot plate. The plate was washed with PBS containing 0.05% Tween 18 hours later, allowed to react with 1 µg/ml biotinized anti-interferon-γ-antibody (7-B6-1; Mabtech) at 37°C for 2.5 hours, washed, and further allowed to react with 1 µg/ml of streptavidin alkaline phosphatase (Mabtech) for 1.5 hours. The plate was washed and then stained with an alkaline phosphatase conjugate substrate kit (BioRad, Hercules, CA, U.S.A.), and the spots were counted under a microscope.

CD4-positive cells were stimulated with mature LCs in the following manner. The mature LCs (2 x 10⁵ cells) and the autologous CD4-positive cells (2 x 10⁵ cells) were cultured in the presence of the anti-CD3 antibody (UCHT1, 0.5 mg/ml; BD PharMingen, SanDiego, CA, U.S.A.) on a 24-well plate, IL-2 (20 IU/ml) was added thereto on day 4 of culture, and the number of CD4-positive cells was counted on day 7. Instead of mature LCs, mature DCs differentiated from CD14-positive monocytes in the presence of GM-CSF, IL-4, CD40 ligands, and TNF-α were used to conduct the same test.

The following results were obtained through Example 2.

### Expression of HES-1 genes induced by Delta-1

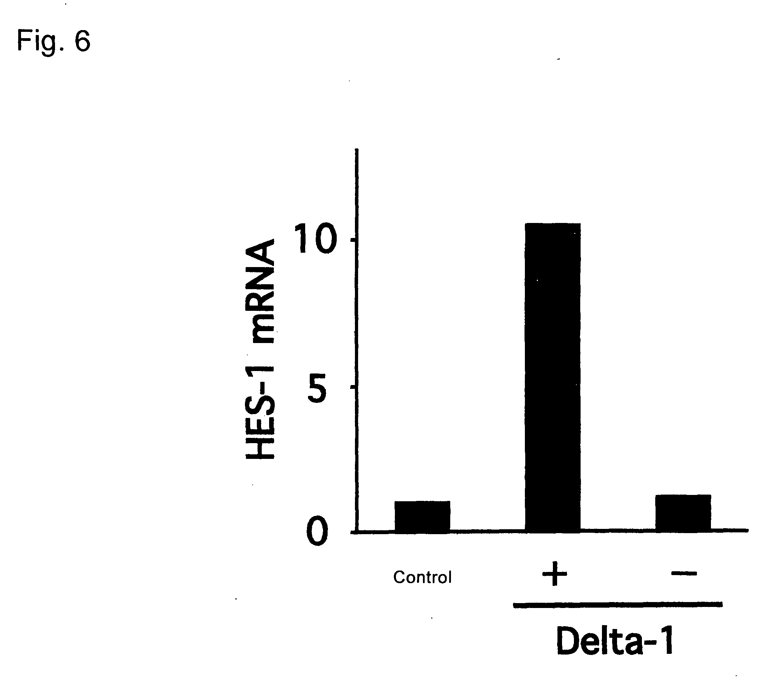

The HES-1 genes are known as the target genes of Notch signal (Schroeter EH et al., Nature 393: 382-386, 1998; Struhl G et al., Cell 93: 649-660, 1998). In order to confirm that Delta-1 transmits Notch signals to LCs, effects of Delta-1 on HES-1 gene expression were examined (Fig. 6). In the culture system containing Delta-1, the expression level of HES-1 genes was as high as approximately 8 times that in the culture system containing no Delta-1. This indicates that Delta-1 affects CD14-positive cells.

Fig. 6 shows the level of HES-1 gene expression enhanced by Delta-1 in CD14-positive cells. The level of HES-1 gene expression in the presence of Delta-1 was compared with that in the absence of Delta-1. The results are represented in terms of the ratio of HES-1 gene expression to GAPDH gene expression, and the expression level was represented while setting the expression level of HES-1 genes in the fresh CD14-positive cells at 1.0. A representative experiment among two experiments is described.

### Electron microscopy

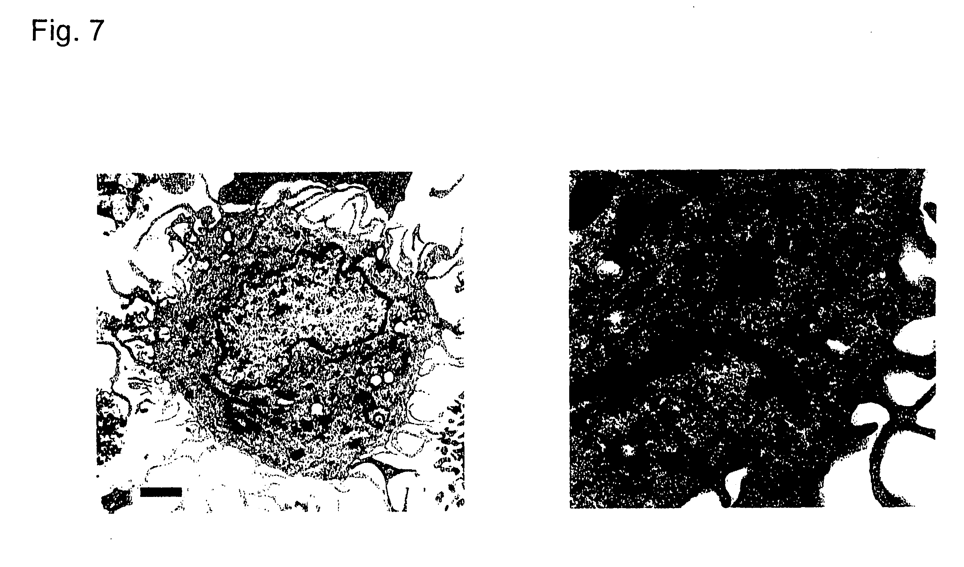

The cells had a large number of elongated processes and contained Birbeck granules in their cytoplasms (Fig. 7).

Fig. 7 is an electron micrograph of cultured cells, wherein the original magnification is x8,000 and a bar is 2 µm in the left panel, and the original magnification is x30,000 and a bar is 0.5 µm in the right panel.

### Phagocytic activity of LCs

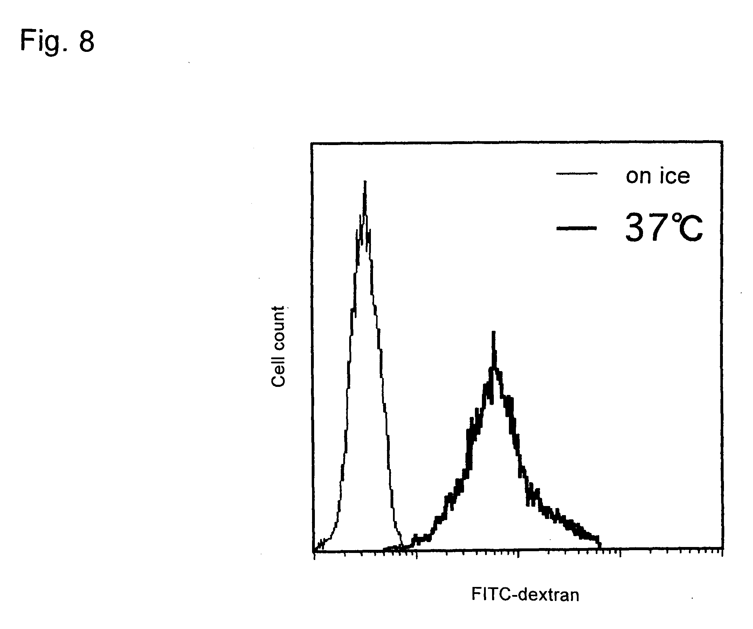

The phagocytic activity of LCs prepared from CD14-positive cells in the presence of Delta-1, GM-CSF, and TGF-β was assayed based on FITC-dextran uptake (Fig. 8). LCs incorporated FITC-dextran. The resulting LCs were found to have phagocytic activity.

Fig. 8 shows the results of analyzing FITC-dextran uptake by LCs. LCs derived from CD14-positive cells in the presence of Delta-1, GM-CSF, and TGF-β were allowed to react with FITC-dextran at 37°C or on ice for 1 hour. LCs were washed 3 times and then analyzed with a FACS Calibur. Similar results were obtained in 3 experiments.

### Maturation of LCs generated in the presence of Delta-1, GM-CSF, and TGF-β

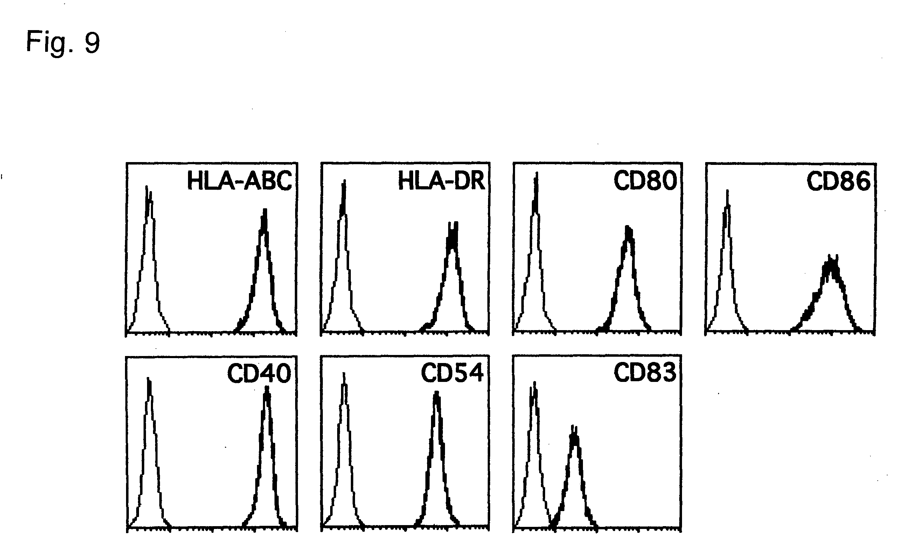

Whether or not LCs generated in the presence of Delta-1, GM-CSF, and TGF-β matured upon reaction with CD40 ligands and TNF-α was inspected (Fig. 9). CD14-positive cells were cultured in the presence of Delta-1, GM-CSF, and TGF-β for 6 days, and during the last 2 days thereof culture was conducted in the presence of CD40 ligands and TNF-α. In the resulting cultured cells, expression levels of HLA-ABC, HLA-DR, CD80, CD86, CD40, and CD54 were enhanced remarkably, and expression of CD83 was also observed. This indicates that LCs generated in the presence of Delta-1, GM-CSF, and TGF-β would mature.

Fig. 9 shows the expression of HLA-ABC, HLA-DR, CD80, CD86, CD40, CD54, and CD83 in LCs matured by CD40 ligand and TNF-α. CD14-positive cells were cultured in the presence of Delta-1, GM-CSF, and TGF-β, CD40 ligands and TNF-α were added on day 4 of culture, and cells were collected on day 6. Expression levels of HLA-ABC, HLA-DR, CD80, CD86, CD40, CD54, and CD83 were analyzed using an FACS Calibur. A bold line indicates the expression of molecules shown in the drawing, and a fine line indicates that of the isotype control. Similar results were obtained in 5 experiments.

### Stimulation of CD8-positive cells with mature LCs derived from CD14-positive cells

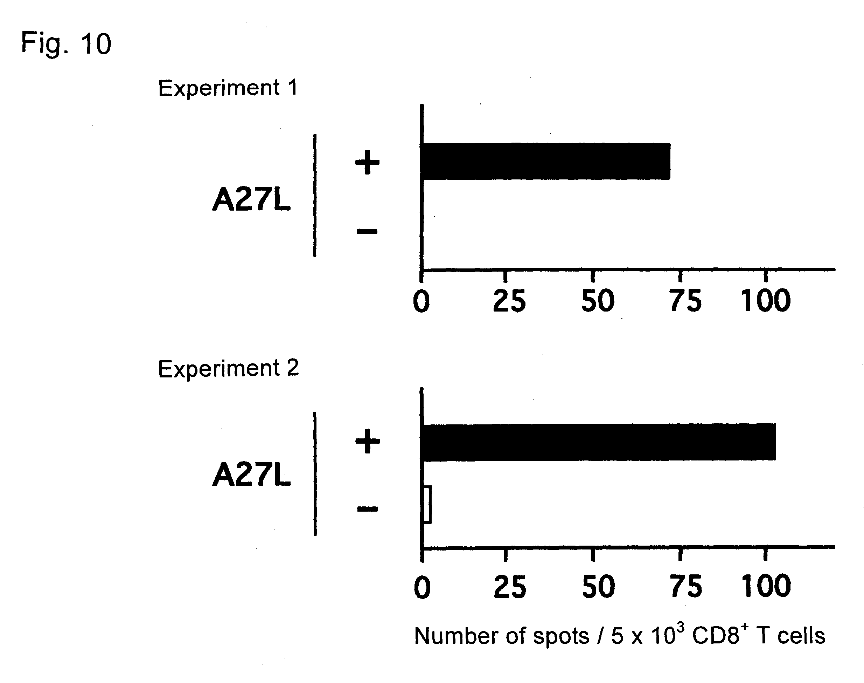

In order to examine functions of mature LCs generated from CD14-positive cells, mature LCs were prepared from the CD14-positive cells obtained from healthy HLA-A0201-positive volunteers. These cells were pulsed with the modified Melan-A₂₆₋₃₅ peptides (A27L), which specifically bind to HLA-A0201. The autologous CD8-positive T cells, which had been cultured together with the peptide-pulsed mature LCs, as effector cells, were allowed to react with the T2 cells, which had been also pulsed with the same modified peptides, as target cells. The number of CD8-positive T cells producing IFN-γ was assayed by the Elispot method. The peptide-pulsed mature LCs induced the expression of the autologous CD8-positive T cells producing IFN-γ (Fig. 10).

As shown in Fig. 10, mature LCs generated from CD14-positive cells activate peptide-specific CD8-positive cells. Mature LCs were prepared from the CD14-positive cells obtained from healthy HLA-A0201-positive volunteers using Delta-1, GM-CSF, TGF-β, CD40 ligand, and TNF-α. The autologous CD8-positive T cells (2 x 10⁵ cells) were stimulated with the mature LCs (4 x 10⁴ cells) that had been pulsed with the modified Melan-A₂₆₋₃₅ peptides (A27L) for 10 days. Thereafter, the CD8-positive cells were collected, and the CD8-positive cells (1 x 10⁴ cells) were cultured together with the T2 cells (5 x 10⁴ cells), which had been pulsed with the modified Melan-A₂₆₋₃₅ peptides (A27L) (ELAGIGILTV) on an Elispot plate. The number of autologous CD8-positive T cells producing interferon-γ was determined 18 hours later. In the drawing, each bar represents an average of 2 measured values. Two experimental operations are described.

### Stimulation of CD4-positive cells with mature LCs derived from CD14-positive cells

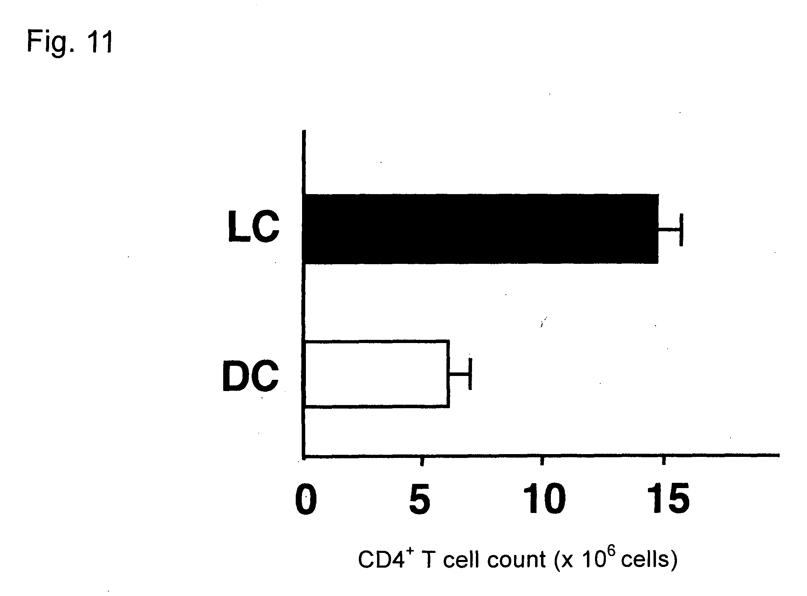

The capacity of mature LCs generated from the CD14-positive cells to stimulate autologous CD4-positive cells was examined. Mature LCs or DCs were cultured together with CD4-positve cells in the presence of an anti-CD3 antibody, and the stimulated CD4-positive cells were amplified with IL-2. The number of CD4-positive cells was determined on day 7 of culture. The number of the CD4-positive cells stimulated with mature LCs was significantly higher than that stimulated with mature DCs (Fig. 11).

Fig. 11 shows the results of stimulation of autologous CD4-positive cells by mature LCs and with mature DCs. Mature LCs were prepared from CD14-positive cells obtained from healthy volunteers using Delta-1, GM-CSF, TGF-β, CD40 ligand, and TNF-α, and mature DCs were prepared therefrom using GM-CSF, IL-4, CD40 ligand, and TNF-α. The autologous CD4-positive T cells (2 x 10⁵ cells) were stimulated with mature LCs or mature DCs (2 x 10⁵ cells). IL-2 was added on day 4 of culture. The number of CD4-positive T cells was counted on day 7. In the drawing, each bar represents an average of 4 measured values. The result is the representative of 2 experiments.

As is shown in the results obtained in Example 2, Delta-1 was found to affect CD14-positive monocytes derived from human peripheral blood based on an increased level of HES-1 gene expression, which is a target gene of Notch signal. As a result of observing the resulting LCs under an electronic microscope, Birbeck granules specific for LCs were found to be generated. Also, these LCs were found to have phagocytic activity. When LCs were allowed to react with CD40 ligand and TNF-α, the expression levels of major histocompatibility complex (MHC) class I (HLA-ABC) and class II (HLA-DR), co-stimulatory molecules CD80 and CD86, and adhesion molecules CD40 and CD54 were significantly increased, and the expression of CD83 was also observed. This indicates that the generated LCs are capable of maturation. With the use of the modified Melan-A₂₆₋₃₅ peptides (A27L) (ELAGIGILTV) derived from the Melan-A molecule, i.e., a cancer-testis antigen, and having a binding motif to HLA-A0201, the capacity of the mature LCs to induce cytotoxic T lymphocytes (CTL) specific for the Melan-A molecules was inspected via the enzyme-linked immunosorbent spot (Elispot) assay. As a result, mature LCs were found to induce the expression of CD8-positive CTL reacting with the Melan-A molecules. The capacity of LCs to stimulate autologous CD4-positive T cells was higher than that of mature dendritic cells (DC), which had been differentiated from CD14-positive monocytes of human peripheral blood in the presence of GM-CSF, interleukin-4 (IL-4), CD40 ligand, and TNF-α.

The present invention is biologically important in terms of the discovery of a novel differentiation pathway of human LCs. Further, development of an effective method of inducing differentiation of LCs from CD14-positive monocytes of human peripheral blood *ex vivo,* which had been difficult in the past, is also important. In conventional techniques of cell therapy using a DC-based vaccine, so-called dermal DCs differentiated from CD14-positive monocytes of human peripheral blood with the aid of GM-CSF, IL-4, and TNF-α are employed. The development of a novel technique for immune-based cell therapy using LCs, which is based on the findings of the present invention, could permit remarkable advancement in vaccine-based cell therapy in the future.

### Industrial Applicability

Through the culturing of human peripheral blood mononuclear cells in the presence of Notch ligand, GM-CSF, and TGF-β, Langerhans cells, which could not been obtained in the past, can be prepared. Such Langerhans cells can be obtained in immature or mature states and can be used for suppression of cancers, infectious diseases, or graft rejection after transplantation of cells, organs, or tissues; for treatment of graft versus host disease; and for treatment of autoimmune diseases or allergic diseases in vaccine-based cell therapy.

The Langerhans cells obtained by the method of the present invention can be applied to cell-biological research concerning Langerhans cells. Also, the Langerhans cells obtained by the method of the present invention can be used for vaccine-based cell therapy. More specifically, such Langerhans cells can be used for suppression of cancers, infectious diseases, or graft rejection after transplantation of cells, organs, or tissues; for treatment of graft versus host disease; and for treatment of autoimmune diseases or allergic diseases.

All publications cited herein are incorporated herein in their entirety. A person skilled in the art would easily understand that various modifications and changes of the present invention are feasible within the technical idea and the scope of the invention as disclosed in the attached claims. The present invention is intended to include such modifications and changes.

## Claims

1. A method for preparing a Langerhans cell, which comprises culturing a human peripheral blood mononuclear cell in the presence of Notch ligand, GM-CSF, and TGF-β.

2. A method for preparing a Langerhans cell, which comprises transducing Notch signaling from Notch of a human peripheral blood mononuclear cell using Notch ligand, and culturing the human peripheral blood mononuclear cell in the presence of GM-CSF and TGF-β.

3. The method for preparing a Langerhans cell according to claim 1 or 2, which is **characterized in that** Notch ligand is immobilized on a culture vessel.

4. The method for preparing a Langerhans cell according to claim 3, wherein Notch ligand constitutes a fusion peptide with another peptide, and an antibody reacting with said other peptide is immobilized on the culture vessel, so that Notch ligand is immobilized on the culture vessel via the bond between said antibody and said other peptide.

5. The method for preparing a Langerhans cell according to claim 4, wherein said other peptide is myc.

6. The method for preparing a Langerhans cell according to any one of claims 1 to 5, wherein Notch ligand is selected from the group consisting of Delta-1, Delta-2, Delta-3, Delta-4, Jagged-1, and Jagged-2.

7. The method for preparing a Langerhans cell according to claim 6, wherein Notch ligand is Delta-1.

8. The method for preparing a Langerhans cell according to claim 1 or 2, wherein the human peripheral blood mononuclear cell is an isolated CD14-positive cell.

9. The method for preparing a Langerhans cell according to any one of claims 1 to 8, which is **characterized in that** E-cadherin, Langerin, and CCR6 are expressed on the surface of the Langerhans cell.

10. The method for preparing a Langerhans cell according to claim 9, which is **characterized in that** MHC class I molecule HLA-ABC, MHC class II molecule HLA-DR, CD80, and CD86 are further expressed on the surface of the Langerhans cell.

11. A method for preparing a Langerhans cell, which further comprises adding at least one selected from the group consisting of CD40 ligand, TNF-α, and LPS to the Langerhans cell prepared by the method according to any one of claims 1 to 10, and culturing the obtained mixture.

12. The method for preparing a Langerhans cell according to any one of claims 1 to 10, wherein the Langerhans cell is immature.

13. The method for preparing a Langerhans cell according to any one of claims 1 to 11, wherein the Langerhans cell is mature.

14. The Langerhans cell prepared by the method according to any one of claims 1 to 13.

15. The Langerhans cell according to claim 14, on the surface of which E-cadherin, Langerin, and CCR6 are expressed.

16. The Langerhans cell according to claim 15, on the surface of which MHC class I molecule HLA-ABC, MHC class II molecule HLA-DR, CD80, and CD86 are further expressed.

17. A Langerhans cell derived from a human peripheral blood mononuclear cell, on the surface of which E-cadherin, Langerin, CCR6, MHC class I molecule HLA-ABC, MHC class II molecule HLA-DR, CD80, and CD86 are expressed.

18. The Langerhans cell according to any one of claims 15 to 17, which is an immature cell.

19. The Langerhans cell according to any one of claims 15 to 17, which is a mature cell.

20. A pharmaceutical composition comprising the Langerhans cell according to any one of claims 14 to 19.

21. The pharmaceutical composition according to claim 20, which is a therapeutic agent for cancers or infectious diseases.

22. The pharmaceutical composition according to claim 21, wherein the Langerhans cell is a mature cell.

23. The pharmaceutical composition according to claim 20, which is used for suppression of graft rejection after transplantation of cells, organs, or tissues: for treatment of graft versus host disease: and for treatment of autoimmune diseases or allergic diseases.

24. The pharmaceutical composition according to claim 23, wherein the Langerhans cell is an immature cell.

25. A method for preparing a Langerhans cell used for treatment of cancers or infectious diseases, which comprises culturing a peripheral blood mononuclear cell collected from a human in the presence of Notch ligand, GM-CSF, and TGF-β.

26. A method for preparing a Langerhans cell used for suppression of cancers, infectious diseases, or graft rejection after transplantation of cells, organs, or tissues; for treatment of graft versus host disease, and for treatment of autoimmune diseases or allergic diseases; wherein said method comprises culturing a peripheral blood mononuclear cell collected from a human in the presence of Notch ligand, GM-CSF, and TGF-β.

27. The method for preparing a Langerhans cell used for treatment of the diseases according to claim 25 or 26, wherein Notch ligand is selected from the group consisting of Delta-1, Delta-2, Delta-3, Delta-4, Jagged-1, and Jagged-2.

28. The method for preparing a Langerhans cell used for treatment of the diseases according to claim 27, wherein Notch ligand is Delta-1.


SEQUENCE LISTING

<110>  KIRIN BEER KABUSHIKI KAISHA

<120>  A method of preparing Langerhans cells

<130>  PH-2211-PCT

<150> JP 2003/277892
<151> 2003-07-22

<160>  7

<170>  PatentIn version 3.1

<210>  1
<211>  21
<212>  DNA
<213>  Artificial

<220>
<223>  primer

<400>  1
tggaaatgac agtgaagcac c                                                21

<210>  2
<211>  20

<212> DNA
<213> Artificial

<220>
<223> primer

<400> 2
gttcatgcac tcgctgaagc 20

<210> 3
<211> 20
<212> DNA
<213> Artificial

<220>
<223> primer

<400> 3
cgcagatgac ggctgcgctg 20

<210> 4
<211> 18
<212> DNA
<213> Artificial

<220>
<223> primer

<400> 4

gaaggtgaag gtcggagt 18

<210> 5

<211> 20

<212> DNA

<213> Artificial

<220>

<223> primer

<400> 5

gaagatggtg atgggatttc 20

<210> 6

<211> 26

<212> DNA

<213> Artificial

<220>

<223> primer

<400> 6

ttgccatcaa tgaccccttc attgac 26

<210> 7

<211> 10

<212> PRT

<213> Artificial

<220>

<223> Synthetic

<400> 7

Glu Leu Ala Gly Ile Gly Ile Leu Thr Val

1               5                   10